# DEMANDE DE BREVET EUROPEEN

(11) **EP 1 629 823 A1**
(43) Date de publication de la demande: **01.03.2006**
(21) Numéro de dépôt: 05291315.9
(22) Date de dépôt: 20.06.2005
(51) Int. Cl.: A61K 8/365, A61K 8/55, C11D 1/37, A61Q 1/14, A61Q 5/02

(54) **Composition de nettoyage moussante**

(30) Priorité: 26.07.2004 FR 0451652
(71) Demandeur: L'OREAL, 75008 Paris (FR)
(72) Inventeur: Aubrun-Sonneville, Odile, 92160 Antony (FR)
(74) Mandataire: Rasson, Catherine

(57) **Abrégé**

La présente demande concerne une composition de nettoyage pour application topique, contenant, dans un milieu aqueux, un système tensioactif comportant au moins un tensioactif choisi parmi les acides alkyl glycol carboxyliques et leurs sels, et au moins un tensioactif choisi parmi les mono-alkylphosphates, les dialkylphosphates, et leurs sels, et ses utilisations notamment dans les domaines cosmétique ou dermatologique, comme produits de nettoyage ou de démaquillage de la peau et/ou du cuir chevelu.

Le tensioactif anionique peut être notamment le lauryl glycol carboxylate de sodium (BEAULIGHT SHAA), ou sa forme acide.

La composition de l'invention a de bonnes propriétés cosmétiques , et la mousse obtenue est fine et elle se rince bien.

## Description

L'invention a pour objet une composition de nettoyage moussante, rinçable à l'eau, et ayant l'aspect d'un gel, comprenant un système tensioactif comportant au moins un tensioactif ionique carboxylique et au moins au moins un tensioactif du type phosphate, ainsi qu'à ses utilisations dans les domaines cosmétique ou dermatologique, notamment comme produit de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Pour le nettoyage de la peau, il est connu d'utiliser des gels aqueux détergents moussants. Leur action nettoyante est apportée par les tensioactifs qu'ils contiennent, ces tensioactifs mettant en suspension les résidus gras et les pigments des produits de maquillage. Ces gels sont efficaces et agréables à utiliser du fait qu'ils donnent une bonne mousse. Lors de l'utilisation d'un gel moussant, on cherche à avoir un gel donnant de bonnes qualités de mousse, c'est-à-dire une mousse se formant facilement et rapidement, abondante, présentant de fines bulles et une bonne tenue (ne coulant pas), qui se rince rapidement sans laisser de film résiduel. De plus, on cherche de plus en plus à avoir des gels confortables, c'est-à-dire qui laisse un bon confort sur la peau après utilisation, qui ne donne donc ni tiraillements ni sensations de sécheresse cutanée.

Les savons (sels d'acides gras) sont généralement utilisés pour obtenir une très bonne qualité de mousse. Les compositions contenant des savons contiennent en général une très forte teneur en savons (20-30%), qui contribue à la fois aux bonnes performances moussantes et à la qualité de texture. Toutefois, les compositions contenant des savons sont moins bien tolérées, particulièrement par les peaux sensibles car elles donnent une sensation de sécheresse et peuvent être irritantes.

D'autres produits moussants sont obtenus avec des tensioactifs synthétiques ioniques ou non ioniques, tels que le Lauryléther sulfate de sodium, les alkylbétaïnes comme la laurylbétaine, les alkylpolyglucosides (APG). Les mousses qu'ils génèrent sont en général grossières et présentent une mauvaise tenue car elles coulent au lieu de bien tenir sur la peau. Une nouvelle famille de tensioactif, les alkyl glycol carboxylates, conduit à des mousses plus denses mais néanmoins grossières.

Il subsiste donc le besoin de réaliser des gels moussants doux, présentant une très bonne qualité de mousse.

La demanderesse a découvert de manière surprenante qu'on pouvait atteindre le but de l'invention et obtenir un gel moussant ayant à la fois de bonnes propriétés cosmétiques (qualités de la mousse et qualité du rinçage) et de bonnes propriétés de tolérance, en utilisant un système tensioactif comprenant au moins un alkylglycol carboxylate et au moins un tensioactif choisi parmi les mono-alkylphosphates, les dialkylphosphates, et leurs sels.

Ainsi, la présente demande a pour objet une composition de nettoyage pour application topique, contenant, dans un milieu aqueux physiologiquement acceptable, un système tensioactif comportant au moins un tensioactif choisi parmi les acides alkyl glycol carboxyliques et leurs sels, et au moins un tensioactif choisi parmi les mono-alkylphosphates, les dialkylphosphates, et leurs sels.

On entend ici par « application topique » une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable. Par ailleurs, on entend par « milieu physiologiquement acceptable », un milieu compatible avec la peau, les lèvres, le cuir chevelu, les cils, les yeux, les ongles et/ou les cheveux. La composition peut constituer notamment une composition cosmétique ou dermatologique.

Par ailleurs, on entend par « milieu aqueux », un milieu comportant une quantité d'eau d'au moins 35 % en poids, allant de préférence de 35 à 98 % en poids et mieux de 40 à 80 % en poids par rapport au poids total de la composition. Le milieu aqueux des compositions moussantes de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

Les compositions de l'invention sont des compositions de nettoyage moussantes et rinçables, utilisables dans le domaine du nettoyage de la peau, des cheveux ou des muqueuses.

La composition obtenue se présente sous forme d'un fluide ou d'un gel. La viscosité des compositions selon l'invention va de préférence de 0,01 à 50 Pa.s, mesurée à 25°C à l'aide du Rheomat RM180 de Rheometric Scientific à 200 rpm (tours par minute), 10 minutes après la mise en rotation du mobile. L'appareil est équipé d'un mobile différent selon les viscosités, par exemple d'un mobile 2 pour les gammes de viscosités inférieures à 0,7 Pa.s, d'un mobile 3 pour les gammes de viscosités de 0,2 à 4 Pa.s, et d'un mobile 4 pour les gammes de viscosités supérieures à 2 Pa.s.

Les compositions de l'invention ont l'avantage d'être très stables et de ne présenter ni déphasage ni phénomènes de recristallisation au stockage de 4°C à 45°C.

Le système tensioactif dans la composition selon l'invention peut être présent en une quantité (en matière active) allant par exemple de 2 à 30 % en poids, de préférence de 3 à 20 % en poids par rapport au poids total de la composition.

Le rapport en poids entre les tensioactifs choisis parmi les acides alkyl glycol carboxyliques et leurs sels, et les tensioactifs choisis parmi les mono-alkylphosphates, les dialkylphosphates, et leurs sels, peut aller de 90/10 à 10/90 et de préférence de 25/75 à 75/25.

### Acides alkyl glycol carboxyliques

La composition selon l'invention comprend au moins un tensioactif choisi parmi les acides alkyl glycol carboxyliques (ou acides 2-(2-Hydroxyalkyloxy acétique), leurs sels et leurs mélanges. Ce sont des tensioactifs anioniques.

Ces tensioactifs peuvent notamment avoir la formule (I) suivante :

R₁-CHOH-CH₂-O-CH₂-COO⁻ X⁺ (I)

dans laquelle R₁ désigne un radical alkyle saturé ou insaturé, linéaire ou ramifié, comportant de 8 à 30 atomes de carbone, et X désigne l'hydrogène ou un cation minéral ou organique tel que ceux issus d'un métal alcalin (par exemple Na+, K+), NH4+, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Les acides hydroxy-2 alkyl carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Parmi les tensioactifs de formule (I), on peut citer notamment le lauryl glycol carboxylate de sodium, commercialisé sous les dénominations BEAULIGHT SHAA® ou BEAULIGHT LCA-25N® par la société SANYO, ou sa forme acide correspondante commercialisée sous la dénomination BEAULIGHT SHAA (Acid Form)® par la société SANYO.

La quantité de tensioactifs de type alkyl glycol carboxylique peut aller par exemple de 1 à 20 % en poids (en matière active), de préférence de 1 à 15 % en poids par rapport au poids total de la composition finale.

### Mono-alkylphosphates et dialkylphosphates

La composition selon l'invention comprend au moins un tensioactif choisi parmi les mono-alkylphosphates, les dialkylphosphates, leurs sels et leurs mélanges. Ce sont aussi des tensioactifs anioniques.

Ces monoalkylphosphates et dialkyl phosphates comportent une ou plusieurs chaînes alkyle linéaires ou ramifiées, aliphatiques et/ou aromatiques, ayant de 8 à 22 atomes de carbone. Dans le mélange de mono- et di-alkylphosphates, la proportion mono/di peut aller de 100/0 à 50/50. Ces phosphates peuvent être neutralisés par des bases organiques ou minérales telles que la potasse, la soude, la triéthanolamine, l'arginine, la lysine et la N- methylglucamine.

Comme tensioactifs du type phosphate utilisables dans la composition de l'invention, on peut citer par exemple le mono-phosphate de lauryle tel que le produit commercialisé sous la dénomination MAP 20® par la société Kao Chemicals, le sel de potassium de l'acide dodécyl-phosphorique tel que le mélange de mono- et di-ester (diester majoritaire) commercialisé sous la dénomination CRAFOL AP-31® par la société Cognis, les monoester et di-ester d'acide octylphosphorique, tel que le mélange commercialisé sous la dénomination CRAFOL AP-20® par la société Cognis, les monoester et de diester d'acide phosphorique de 2-butyloctanol éthoxylé (7 moles d'OE), tel que le mélange commercialisé sous la dénomination ISOFOL 12 7 EO-PHOSPHATE ESTER® par la société Condea, les sels de potassium ou de triéthanolamine de mono-alkyl (C12-C13) phosphate, tels que le produit commercialisé sous les références ARLATONE MAP 230K-40® et ARLATONE MAP 230T-60® par la société Uniqema, le lauryl phosphate de potassium tel que le produit en solution aqueuse à 40 %, commercialisé sous la dénomination DERMALCARE MAP XC-99/09® par la société Rhodia Chimie, et les mélanges de ces tensioactifs.

La quantité de tensioactif(s) du type phosphate peut aller par exemple de 0,5 à 10 % en poids de matière active, et de préférence de 0,5 à 7 % en poids de matière active, par rapport au poids total de la composition.

### Tensioactifs additionnels

Le système tensioactif peut comporter, outre les tensioactifs indiqués ci-dessus, un ou plusieurs autres tensioactifs additionnels. Toutefois, ces autres tensioactifs s'ils sont présents doivent être en une quantité inférieure à 50% en poids par rapport à la quantité totale de système tensioactif. Ces tensioactifs peuvent être présents par exemple en une quantité (en matière active) allant de 0,5 à 10 % en poids et de préférence de 1 à 7 % en poids par rapport au poids total de la composition.

Ces tensioactifs additionnels peuvent être choisis parmi les tensioactifs non ioniques, amphotères, anioniques et leurs mélanges.

### - Tensioactifs non ioniques

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la société Seppic ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

### - Tensoactifs amphotères

Les tensioactifs amphotères et zwitterioniques peuvent être choisis par exemple parmi les dérivés bétaïnes, amphoacétates, hydroxylsultaines, et leurs mélanges.

Comme bétaïnes, on peut citer par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyéthylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom CTFA : sodium cocamphoacetate).

Comme hydroxylsultaïnes, on peut citer la Cocamidopropyl hydroxysultaïne comme le produit commercialisé sous la dénomination REWOTERIC AM CAS par la société Golschmidt-Degussa.

### - Tensioactifs anioniques :

On peut utiliser par exemple comme tensioactifs anioniques, les carboxylates, les alkylsulfates, les sulfonates, les sulfosuccinates, les alkylsulfoacétates, les phosphates, les polypeptides, les dérivés anioniques d'alkylpolyglucosides, et leurs mélanges.

Comme carboxylates, on peut citer :
- Les amido éthercarboxylates (AEC), comme le Lauryl amido ether carboxylate de sodium (3 OE), commercialisé sous la dénomination AKYPO FOAM 30® par la société Kao Chemicals.
- les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX® par la société Nikkol.
- les sels alcalins de N-acylaminoacides,
- les sarcosinates, comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN ® par la société Nikkol.
- les alaninates, comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA ® par la société Kawaken.
- Les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
- les aspartates, comme le mélange de N-lauroyl aspartate de triethanolamine /N-myristoyl aspartate de triethanolamine commercialisé sous la dénomination ASPARACK ® par la société Mitsubishi.
- Les glycinates, comme le N-cocoyl glycinate de sodium commercialisé sous les dénominations AMILITE GCS-12® et AMILITE GCK 12 par la société Ajinomoto.
- Les citrates tels que le mono-ester citrique d'alcools de coco oxyéthylénés (9 moles), commercialisé sous la dénomination WITCONOL EC 1129 par la société Goldschmidt.
- les galacturonates tels que le dodeécyl d-galactoside uronate de sodium commercialisé par la société Soliance.
- les sels d'acides gras (savons) ayant une chaîne alkyl en C6 à C22, neutralisés par une base organique ou minérale telle que la potasse, la soude, la triéthanolamine,la N-methyl glucamine, la lysine et l'arginine.

Comme alkyl sulfates oxyéthylénés ou non, ton peut citer par exemple le Lauryl éther sulfate de sodium (C12-C14 70/30) (2,2 OE) commercialisé sous la dénomination SIPON AOS 225® par la société Cognis, le Lauryl éther sulfate d'ammonium (C12-C14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le m2lange de lauryl- et oleyl- éther sulfate de sodium et magnésium, commercialisé sous la dénomination EMPICOL BSD 52 par la société Albright & Wilson.

Comme sulfonates, on peut citer par exemple :
- Les alpha oléfines sulfonates tels que l'alpha oléfine sulfonate de sodium (C14-16) commercialisé sous les dénominations BIO-TERGE AS-40® et BIO-TERGE AS-40 CG® par la société Stepan, ou commercialisé sous la dénomination WITCONATE AOS PROTEGE®, SULFRAMINE AOS PH 12® par la société Witco, l'oléfine sulfonate de sodium secondaire commercialisé sous la dénomination HOSTAPUR SAS 30® par la société Clariant.
- Les iséthionates tels que le Cocoyl isethionate de sodium commercialisé sous la dénomination JORDAPON CI P® par la société JORDAN.
- Les taurates tels que le sel de sodium du methyltaurate d'huile de palmiste commercialisé sous la dénomination HOSTAPON CT PATE® par la société Clariant, le N-cocoyl N- methyltaurate de sodium commercialisé sous la dénomination HOSTAPON LT-SF® par la société Clariant ou NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyethylene (3 OE) commercialisé sous la dénomination SETACIN 103 SPECIAL® par la société Zschimmer Schwarz,, commercialisé sous la dénomination REWOPOL SB-FA 30 K 4® par la société Witco, le sel disodique d'un hemi-sulfosuccinate des alcools C12-C14 commercialisé sous la dénomination SETACIN F SPECIAL PASTE® par la société Zschimmer Schwarz, l'oleamido sulfosuccinate di-sodique oxyéthyléné (2 OE) commercialisé sous la dénomination STANDAPOL SH 135® par la société Cognis, le monosulfosuccinate d'amide laurique oxyéthyléné (5 OE) commercialisé sous la dénomination LEBON A-5000® par la société SANYO.

Comme alkyl sulfoacétates, on peut citer par exemple le mélange de lauryl sulfoacetate de sodium, et le lauryl ether sulfosuccinate di-sodique, commercialisé sous la dénomination STEPAN-MILD LSB par la société Stepan.

Comme polypeptides, on peut citer par exemple ceux obtenus par condensation d'une chaîne grasse sur les aminoacides du blé et de l'avoine, tels que le sel de potassium de la lauroyl protéine de blé hydrolysée commercialisé sous la dénomination AMINOFOAM W OR® par la société Croda, la Cocoyl protéine de soja hydrolysée, le sel de tri-éthanolamine commercialisé sous la dénomination MAY-TEIN SY® par la société Maybrook, le sel de sodium de lauroyl amino acides d'avoine commercialisé sous la dénomination PROTEOL OAT® par la société Seppic, l'hydrolysat de collagène greffé sur acide gras de coprah commercialisé sous la dénomination GELIDERM 3000® par la société Deutsche Gelatine, les protéines de soja acylées par des acides de coprah hydrogénés, commercialisées sous la dénomination PROTEOL VS 22® par la société Seppic.

Comme dérivés anioniques d'alkyl polyglucoside, on peut citer les citrates, tartrates, sulfosuccinates, carbonates et éthers de glycérol fabriqués à partir des alkyl polyglucosides, comme le sel de sodium d'ester tartrique de cocoyl polyglucoside (1.4) commercialisé sous la dénomination EUCAROL AGE-ET® par la société Cesalpinia, le sel di-sodique d'ester sulfosuccinique de cocoyl polyglucoside (1.4) commercialisé sous la dénomination ESSAI 512 MP® par la société Seppic, le sel de sodium d'ester citrique de cocoyl polyglucoside (1.4) (1.4) commercialisé sous la dénomination EUCAROL AGE-EC® par la société Cesalpinia, le lauryl polyglucoside éther carboxylate de sodium commercialisé sous la dénomination PLANTAPON LGC SORB par la société Cognis.

Selon un mode particulier de réalisation de l'invention, le système tensioactif comprend outre les tensioactifs de type alkylglycol carboxylate et de type alkylphosphate, au moins un tensioactif non ionique, notamment choisi parmi les alkylpolyglucosides.

### Additifs

La composition de l'invention peut contenir tous les additifs ou actifs classiquement utilisés dans les produits de nettoyage. On peut citer par exemple les conservateurs ; les séquestrants (EDTA) ; les antioxydant ; les parfums ; les matières colorantes telles que les colorants solubles, les pigments, les nacres ; les charges minérales ou organiques, apportant de la viscosité, matifiantes, blanchissantes ou exfoliantes ; les filtres solaires ; les actifs cosmétiques ou dermatologiques, hydrophiles ou lipophiles, tels que les vitamines hydrosolubles ou liposolubles, les antiseptiques, les antiséborrhéïques, les antimicrobiens tels que le peroxyde de benzoyle, l'acide salicylique, le triclosan, l'acide azélaïque, la niacinamide (vit. PP), et aussi les azurants optiques ; les polymères non ioniques, anioniques, cationiques et/ou amphotères ; les corps gras incompatibles avec le milieu aqueux, comme les huiles ou les cires ; les agents régulateurs de viscosité et agents épaississants ; ou d'autres agents ayant pour effet d'améliorer les propriétés cosmétiques de cheveux ou de la peau. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

La composition de l'invention peut notamment contenir des agents régulateurs de viscosité et agents épaississants dont la quantité dépend de la viscosité souhaitée pour la composition finale. Ces agents peuvent être présents à des concentrations allant de préférence de 0,05 à 10 % en poids et de préférence allant de 0.05 à 5 % en poids par rapport au poids total de la composition.

Comme agents régulateurs de viscosité et agents épaississants, on peut citer les électrolytes ; les scléroglucanes ; les polymères épaississants ; les particules ; les amides grasses ; et leurs mélanges.

Les polymères épaississants peuvent être anioniques, amphotères, cationiques ou non ioniques, réticulés ou non, modifiés hydrophobes ou non, naturels ou de synthèse.

Comme polymères épaississants, on peut utiliser par exemple des polymères dérivés d'acide carboxylique, de polyacrylamide et/ou d'acide acrylamido-2-méthyl propane sulfonique. Le ou les polymères dérivés carboxyliques peuvent des polymères associatifs (c'est-à-dire comportant un groupement hydrophobe) ou non associatifs, solubles ou dispersibles dans l'eau, gonflants en milieu alcalin ou non. Ils peuvent être sous forme de poudre, de latex, en émulsion ou dispersés dans l'eau. Les polymères peuvent être non ioniques, anioniques, cationiques, zwitterioniques ou amphotères. Les monomères présents dans les polymères sont choisis de préférence parmi les monomères styrène, butadiène, éthylène, acrylonitrile, chloroprène, chlorure de vinylidène, isoprène, isobutylène, chlorure de vinyle, et esters d'acides acrylique, methacrylique, vinylacétique, maléique, crotonique et itaconique. Ces monomères peuvent être utilisés seuls ou en combinaison ou peuvent être mélangés avec un ou plusieurs monomères ioniques comme par exemple les acides acryliques ou methacryliques sous forme chargée.

Les polymères anioniques préférés contiennent un monomère dérivé d'acide acrylique ou méthacrylique et sont partiellement neutralisés comme par exemple les polymères commercialisés sous les dénominations Carbopol 981 et Carbopol 1382 par la société Noveon, et le polymère commercialisé sous al dénomination Acrysol 22 par la société Röhm & Haas.

Comme polymères épaississants non ioniques, on peut utiliser des dérivés oxyalkylénés d'esters d'acide gras ou d'éthers d'alcools gras, ou des polysaccharides telles que la gomme de xanthane. On peut citer comme dérivés oxyalkylénés d'esters d'acide gras ou d'éthers d'alcools gras, notamment les dérivés alkyl ou acyl éthoxylés de polyols, qui peuvent être en particulier des dérivés oxyéthylénés d'esters d'acide gras en C6-C30 ou d'éthers d'alcool gras en C6-C30, et de polyols tels que glycérol, sorbitol, glucose, pentaerythritol, ces dérivés oxyéthylénés comportant généralement de 50 à 500 groupes oxyéthylénés et de préférence de 100 à 300 groupes oxyéthylénés. Comme composés de ce type, on peut citer par exemple le stéarate de glycéryle oxyéthyléné (200 OE) tel que le produit commercialisé sous la dénomination SIMULSOL 220 TM ® par la société Seppic, le tétrastéarate de pentaérythrityle oxyéthyléné (150 OE) tel que le produit commercialisé sous la dénomination CROTHIX ® par la société Croda, le di-oléate de méthylglucose oxyéthyléné (120 OE) tel que le produit commercialisé sous la dénomination GLUCAMATE DOE-120 VEGETAL® par la société Amerchol, le triisostéarate de sorbitan oxyéthyléné (160 OE) tel que le produit commercialisé sous la dénomination RHEODOL TW IS399C par la société Kao Chemicals, et leurs mélanges. Comme polysaccharides, on peut citer le Cetyl hydroxyethylcellulose commercialisé sous la dénomination Natrosol plus grade 330 CS par la société Hercules, et leurs mélanges.

De manière préférée, les polymères épaississants sont choisis parmi les polymères non ioniques et anioniques.

La composition selon l'invention est de préférence exempte de polysaccharides cationiques tels que les gommes de guar ou de galactomannane cationiques. Toutefois, elle peut contenir des polymères cationiques autres que les tels que les polysaccharides cationiques, comme par exemple ceux dérivés d'acrylamide, d'acrylate ou de chlorure de dimethyldiallyl ammonium.

Comme particules épaississant les formules, on peut utiliser les argiles comme les hectorites telles que la Bentone MA commercialisée par la société Elementis Specialities.

Comme amides grasses, on peut utiliser par exemple la cocamide MEA, la cocamide MIPA.

Comme électrolytes, on peut utiliser par exemple le chlorure de sodium, le chlorure de potassium, et des sels analogues.

Les compositions selon l'invention peuvent avoir une apparence allant du produit fluide à un gel. Elles sont stables et ont une très bonne rinçabilité. Elles peuvent constituer par exemple un produit de nettoyage et/ou de démaquillage de la peau, du cuir chevelu et/ou des cheveux, un produit de gommage et/ou un produit exfoliant pour la peau. Elles peuvent constituer plus particulièrement une composition de nettoyage de la peau.

Un autre objet de l'invention consiste en l'utilisation cosmétique de la composition telle que définie ci-dessus, comme produit de nettoyage et/ou de démaquillage de la peau, du cuir chevelu et/ou des cheveux, et/ou comme produit de gommage et/ou produit exfoliant pour la peau.

Les compositions selon l'invention peuvent être utilisées de deux façons :
- la première utilisation consiste à étaler le gel dans les mains, à l'appliquer sur le visage ou sur le corps puis à le masser en présence d'eau pour développer la mousse directement sur le visage ou le corps.
- l'autre utilisation possible de ce type de produit consiste à développer la mousse dans les paumes des mains avant de l'appliquer sur le visage ou le corps.
Dans les deux cas, la mousse est ensuite rincée.

Les compositions selon l'invention peuvent constituer aussi une composition pour le traitement des peaux grasses, notamment quand elles contiennent un actif spécifique de traitement des peaux grasses tels que, par exemple, l'acide salicylique, l'acide azélaïque, le triclosan, le piroctone olamine, la niacinamide (vitamine PP).

Un autre objet de l'invention est l'utilisation de la composition telle que définie ci-dessus pour la préparation d'une composition destinée à traiter la peau grasse.

Les exemples qui suivent servent à illustrer l'invention sans toutefois présenter un caractère limitatif. Les quantités indiquées sont en % en poids sauf mention contraire.

Dans le tableau ci-dessous, tous les pourcentages sont exprimés en poids de matière active (M.A.)

### Performances sensorielles :

Les performances sensorielles des compositions (qualités de mousse) ont été déterminées selon le protocole décrit ci-dessous.

Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- ajouter 2 ml d'eau et travailler le produit à nouveau pendant 10 secondes,
6- évaluer la qualité de la mousse selon les critères définis ci-dessous,
7- rincer les mains sous l'eau,
8- les essuyer.

Les critères de mousse sont notés sur une échelle de 0 à 10.
- étapes 4-6 : évaluation de la qualité de mousse
- *Le volume de mousse :* la note attribuée est d'autant plus élevée que le volume est grand.
- *La taille des bulles composant la mousse :* la note attribuée est d'autant plus élevée que les bulles sont grosses.
- *La tenue de la mousse :* la note attribuée est d'autant plus élevée que la mousse est élastique et ne coule pas
- étape 7 : évaluation pendant le rinçage
- *Le rinçage :* la note attribuée est d'autant plus faible que la présence d'un film glissant difficile à éliminer est grande, ce qui signifie que la note est d'autant plus forte que le produit se rince facilement sans laisser de film.

Le panel d'évaluation est constitué de 4 experts entraînés. La moyenne des quatre notes permet de comparer les compositions selon chacun des critères.

**Exemples 1 et 2 selon l'invention et exemples comparatifs 1 et 2**

| Composition | Exemple 1 selon l'invention | Exemple 2 selon l'invention | Exemple comparatif 1 | Exemple comparatif 2 |
|---|---|---|---|---|
| Sodium Lauryl Glycol Carboxylate (1) | 7,8 | 11,7 | 15,6 | 7,8 |
| Monoalkyl phosphate (2) | 6,5 | 3,25 | - | |
| Sodium Laureth sulfate (3) | - | - | - | 6,51 |
| Potasse | 1,7 | 0,85 | - | |
| Acide citrique | - | - | 0,035 | |
| Imidazolidinyl urée | - | - | 0,2 | |
| Eau | Qsp100 | Qsp100 | Qsp100 | |
| | | | | |
| pH | 7,2 | 7 | 7,35 | 7 |
| Taille de bulles | 3,6 | 4 | 4,8 | 5 |
| Volume de mousse | 6,8 | 6,8 | 6,9 | 6,8 |
| Tenue de la mousse | 7,3 | 7,5 | 7,5 | 7,8 |
| Rinçage | 9,1 | 9 | 8,5 | 8 |

| | | | | |
|---|---|---|---|---|
| (1) Beaulight Shaa (SANYO) à 30 % de matière active, introduit en une quantité adéquate pour avoir le % de matière active indiquée dans le tableau | | | | |
| (2) MAP 20 (KAO) (100 % de matière active) | | | | |
| (3) EMPICOL ESB 70/FL 2 (HUNSTMAN) à 70 % en matière active, introduit en une quantité adéquate pour avoir le % de matière active indiquée dans le tableau | | | | |

Les exemples réalisés montrent que l'association lauryl glycol carboxylate /alkylphosphate permet d'obtenir des mousses plus fines, tout en gardant un bon volume de mousse avec une densité élevée, ainsi qu'un bon rinçage. En revanche, l'association du lauryl glycol carboxylate avec un autre tensioactif anionique comme le lauryléther sulfate ne permet pas de réduire la taille des bulles comme le montre l'exemple comparatif 2.

**Exemple 3 selon l'invention et exemple comparatif 3**

| Composition | Exemple 3 selon l'invention | Exemple comparatif 2 |
|---|---|---|
| Sodium Lauryl Glycol Carboxylate (1) | 5,2 | 7,8 |
| Monoalkyl phosphate (2) | 4,33 | - |
| Sodium Laureth sulfate (3) | - | - |
| Décyl glucoside (4) | 4,33 | 6,5 |
| Potasse | 1,13 | - |
| Acide citrique | - | 0,02 |
| Imidazolidinyl urée | - | - |
| Eau | Qsp100 | Qsp100 |
| | | |
| pH | 6,85 | 7,23 |
| Taille de bulles | 3,9 | 5,4 |
| Volume de mousse | 5,4 | 8,4 |
| Tenue de la mousse | 7,1 | 8,3 |
| Rinçage | 9 | 8,9 |

| | | |
|---|---|---|
| (1) Beaulight Shaa (SANYO) à 30 % de matière active, introduit en une quantité adéquate pour avoir le % de matière active indiquée dans le tableau | | |
| (2) MAP 20 (KAO) (100 % de matière active) | | |
| (3) EMPICOL ESB 70/FL 2 (HUNSTMAN) à 70 % en matière active, introduit en une quantité adéquate pour avoir le % de matière active indiquée dans le tableau | | |
| (4) MYDOL10 (KAO) à 40 % de matière active, introduit en une quantité adéquate pour avoir le % de matière active indiquée dans le tableau | | |

Les exemples réalisés montrent que l'association lauryl glycol carboxylate /alkylphosphate permet d'obtenir des mousses plus fines, tout en gardant un bon rinçage.

## Revendications

1. Composition de nettoyage pour application topique, contenant, dans un milieu aqueux physiologiquement acceptable, un système tensioactif comportant au moins un tensioactif choisi parmi les acides alkyl glycol carboxyliques et leurs sels, et au moins un tensioactif choisi parmi les mono-alkylphosphates, les dialkylphosphates, et leurs sels.

2. Composition selon la revendication 1, **caractérisée en ce que** le système tensioactif est présent en une quantité allant de 2 à 30 % en poids par rapport au poids total de la composition.

3. Composition selon la revendication 1 ou 2, **caractérisée en ce que** le rapport en poids entre les tensioactifs choisis parmi les acides alkyl glycol carboxyliques et leurs sels, et les tensioactifs choisis parmi les mono-alkylphosphates, les dialkylphosphates, et leurs sels, va de 90/10 à 10/90 et de préférence de 25/75 à 75/25.

4. Composition selon l'une quelconque des revendications précédentes, **caractérisée en ce que** le tensioactif choisi parmi les acides alkyl glycol carboxyliques et leurs sels, a la formule (I) suivante :
R₁-CHOH-CH₂-O-CH₂-COO⁻ X⁺ (I)
dans laquelle R₁ désigne un radical alkyle saturé ou insaturé, linéaire ou ramifié, comportant de 8 à 30 atomes de carbone, et X désigne l'hydrogène ou un cation minéral ou organique.

5. Composition selon la revendication précédente, **caractérisée en ce que** le tensioactif choisi parmi les acides alkyl glycol carboxyliques et leurs sels est un composé de formule (I) dans laquelle R₁ désigne un radical alkyle saturé ou insaturé, linéaire ou ramifié comportant de 8 à 18 atomes de carbone.

6. Composition selon la revendication précédente, **caractérisée en ce que** le tensioactif choisi parmi les acides alkyl glycol carboxyliques et leurs sels est le lauryl glycol carboxylate de sodium, ou sa forme acide.

7. Composition selon l'une quelconque des revendications précédentes, **caractérisée en ce que** la quantité de tensioactif(s) choisi(s) parmi les acides alkyl glycol carboxyliques et leurs sels va de 1 à 20 % en poids par rapport au poids total de la composition.

8. Composition selon l'une quelconque des revendications précédentes, **caractérisée en ce que** le tensioactif choisi parmi les mono-alkylphosphates, les dialkylphosphates et leurs sels, est choisi parmi le mono-phosphate de lauryle, le sel de potassium de l'acide dodécyl-phosphorique, le mélange de monoester et de di-ester d'acide octylphosphorique, le mélange de monoester et de diester d'acide phosphorique de 2-butyloctanol éthoxylé (7 moles d'OE), le sel de potassium ou de triéthanolamine de mono-alkyl (C12-C13) phosphate, le lauryl phosphate de potassium en solution aqueuse à 40 %, et leurs mélanges.

9. Composition selon l'une quelconque des revendications précédentes, **caractérisée en ce que** la quantité de tensioactif(s) choisi(s) parmi les mono-alkylphosphates, les dialkylphosphates, leurs sels, va de 0,5 à 10 % en poids par rapport au poids total de la composition.

10. Composition selon l'une quelconque des revendications précédentes, **caractérisée en ce que** le système tensioactif comprend, en outre, un ou plusieurs tensioactifs additionnels non ioniques, amphotères ou anioniques.

11. Composition selon la revendication précédente, **caractérisée en ce que** le tensioactif additionnel est un tensioactif non ionique choisi parmi les alkylpolyglucosides.

12. Composition selon l'une quelconque des revendications précédentes, **caractérisée en ce qu'**elle comprend un agent épaississant choisi parmi les polymères épaississants, les particules, les amides grasses, les électrolytes, et leurs mélanges.

13. Utilisation cosmétique d'une composition selon l'une quelconque des revendications 1 à 12, comme produit de nettoyage et/ou de démaquillage de la peau, du cuir chevelu et/ou des cheveux, et/ou comme produit de gommage et/ou produit exfoliant pour la peau.

14. Utilisation de la composition selon l'une quelconque des revendications 1 à 12, pour la préparation d'une composition destinée à traiter la peau grasse.
